# EUROPEAN PATENT SPECIFICATION

(11) **EP 2 995 954 B1**
(45) Date of publication and mention of the grant of the patent: **24.11.2021**
(21) Application number: 14794167.8
(22) Date of filing: 09.05.2014
(51) Int. Cl.: G01N 21/64, G01N 33/58, G01N 33/533

(54) **PREPARATION OF VISIBLE COLORED INSOLUBLE CARRIER PARTICLES LABELED WITH A FLUORESCENT DYE AND AN IMMUNOASSAY METHOD USING THE SAME**
HERSTELLUNG VON FARBIGEN, UNLÖSLICHEN TRÄGERTEILCHEN, DIE MIT EINEM FLUORESZENZ-FARBSTOFF MARKIERT SIND UND IMMUNOASSAY-VERFAHREN UNTER VERWENDUNG DIESER TEILCHEN
PRÉPARATION DE PARTICULES SUPPORT NON SOLUBLES COLORÉES QUI SONT MARQUÉES AVEX UN COLORANT FLUORESCENT, ET PROCÉDÉ DE DOSAGE IMMUNOLOGIQUE UTILISANT LESDITES PARTICULES

(30) Priority: 10.05.2013 JP 2013100422
(43) Date of publication of application: 16.03.2016
(73) Proprietor: Denka Company Limited, Tokyo 103-8338 (JP)
(72) Inventor: MIYAZAWA Takashi, Gosen-shi Niigata 959-1695 (JP)
(74) Representative: Wohlfahrt, Jan Günther
(86) International application number: PCT/JP2014/062462
(87) International publication number: WO 2014/181863

(56) References cited:
- EP-A1- 3 029 462
- JP-A- 2006 170 658
- JP-A- 2010 014 631
- JP-A- 2010 145 272
- JP-A- 2011 220 705
- JP-A- 2012 242 162
- US-A1- 2009 289 201
- None

## Description

### Technical Field

The present invention relates to a method for detecting an analyte contained in a sample in a specific and rapid manner and a kit using the method.

### Background Art

Hitherto, immunoassay methods using insoluble carrier particles have been widely performed.

Immunoassay methods using insoluble carrier particles comprise the following steps: preparing a test piece on which a ligand capable of specifically binding to a target substance has been immobilized at a certain site on a membrane having a fiber structure such as a nitrocellulose membrane; allowing insoluble carrier particles (e.g., gold colloid, platinum-gold colloid, and/or colored polystyrene particles) on which a ligand capable of specifically binding to a target substance has been immobilized and a sample containing a target substance to be developed on the test piece with the use of a capillary phenomenon, thereby causing the target substance to be sandwiched between the ligand on the nitrocellulose membrane and the ligand on the re-dissolved insoluble carrier particles; and detecting the target substance by visual observation or with the use of a dedicated apparatus.

Insoluble carrier particles (e.g., gold colloid, platinum-gold colloid, and colored polystyrene particles) are advantageous in that they can be detected by visual observation. However, detection sensitivity would not be drastically improved even by applying these insoluble carrier particles to a dedicated apparatus for detection. Meanwhile, when a ligand labeled with a fluorescent substance or insoluble carrier particles containing a fluorescent substance are used, detection sensitivity can be drastically improved with the use of a dedicated apparatus, which is advantageous. In this case, however, such ligand or particles cannot be detected by visual observation, and large amounts of target substances are necessary. In addition, even if detection can be carried out within a short period of time upon visual observation, a dedicated apparatus needs to be used, which disadvantageously results in complicated detection procedures.

In order to solve the above problem, the use of a mixture of insoluble carrier particles that allow visual observation and fluorescent particles has been suggested (see Patent Literature 1). In this case, however, ligands on insoluble carrier particles and ligands on fluorescent particles compete with each other. Therefore, if the amount of a target substance is very small, ligands on fluorescent particles cannot bind to the target substance, and this does not always result in high sensitivity. In addition, in consideration of production efficiency, two types of insoluble carrier particles must be separately prepared. This is problematic due to poor efficiency.

Further, the use of composite particles for immunochromatography, the particles being characterized in that the exteriors of fine metal particles are covered with at least one layer of silica containing at least one type of fluorescent substance, has been suggested (see Patent Literature 2). This invention is based on the premise that light absorption by fine metal particles causes absorption of fluorescence from the fluorescent substance on the exterior side, which means that the type of fluorescent substance is limited and the use of a silica layer is necessary. In this case, special techniques are required to prepare such particles, which is also problematic. US 2009/289201 A1 (Patent Literature 3) discloses fluorescent dye tagged colored latex beads wherein the bead color is the same as the color emitted by the fluorescent tag.

### Citation List

### Patent Literature

Patent Literature 1: JP Patent Publication (Kokai) No. 2010-014631 A
Patent Literature 2: JP Patent Publication (Kokai) No. 2011-220705 A
Patent Literature 3: US Patent Publication US 2009/289201 A1

### Summary of Invention

### Technical Problem

Insoluble carrier particles used in immunoassay methods have been widely available as *in vitro* diagnostic products for point of care test (POCT), and they have been used particularly for diagnostic aids for bacterial or viral infectious diseases. Such infectious diseases are sometimes lifethreatening when affecting infants and elderly people with compromised immunity. Influenza virus infectious diseases and the like are also lifethreatening for children and adults. Meanwhile, there are antibiotics and specific medicines for certain bacteria and viruses, meaning that early diagnosis can result in reliable treatment. In addition, influenza virus infectious diseases are highly infectious and thus may cause transmission of infections in hospitals and clinic waiting rooms. Under the present circumstances, *in vitro* diagnostic products for POCT are required to be highly sensitive and enable determination within a shortest possible period of time.

The object of the present invention is to provide: insoluble carrier particles used for high-sensitivity rapid immunoassay, which allow visual observation and high-sensitivity apparatus measurement; a method for preparing the same; an immunoassay method using the same; and an immunoassay kit including the same.

### Solution to Problem

It is possible to solve the above problem by binding a fluorescent substance to a functional carboxyl group on a visible colored insoluble carrier particle which is a colored polystyrene latex particle that allows visual confirmation when a ligand is bound thereto. A combination of insoluble carrier particles and a fluorescent substance that emits fluorescence having a wavelength of a color complementary to a color having a wavelength equivalent to the absorption wavelength of the insoluble carrier particles allows efficient detection without causing absorption of fluorescence by the insoluble carrier particles. Specifically, a fluorescent substance that emits red fluorescence is used for red insoluble carrier particles. The use of such combination for immunoassay allows rapid detection based on visual observation when the amount of a target substance is large and it also allows high-sensitivity detection with the use of a dedicated apparatus when the amount of a target substance is small.

The invention is defined by the claims. Any subject-matter falling outside the scope of the claims is provided for information purposes only. Specifically, the present disclosure further encompasses the following.

[1] Visible colored insoluble carrier particles labeled with a fluorescent dye, which are used for immunoassay, wherein absorption of fluorescence by the visible colored insoluble carrier particles is low within a wavelength range of fluorescence emitted by the fluorescent dye.
[2] The visible colored insoluble carrier particles labeled with a fluorescent dye according to [1], wherein the fluorescent dye used for labeling is a fluorescent substance that emits fluorescence with a wavelength of a color complementary to a color with a wavelength equivalent to an absorption wavelength of the visible colored insoluble carrier particles.
[3] The visible colored insoluble carrier particles labeled with a fluorescent dye according to [1] or [2], wherein the visible colored insoluble carrier particles are blue-colored particles and the wavelength of fluorescence emitted by the fluorescent dye is 450 nm - 570 nm.
[4] The visible colored insoluble carrier particles labeled with a fluorescent dye according to [3], wherein the visible colored insoluble carrier particles are blue-colored particles and the fluorescent dye is fluorescein or fluorescein isothiocyanate.
[5] The visible colored insoluble carrier particles labeled with a fluorescent dye according to [1] or [2], wherein the visible colored insoluble carrier particles are red-colored particles and the wavelength of fluorescence emitted by the fluorescent dye is 590 nm - 750 nm.
[6] The visible colored insoluble carrier particles labeled with a fluorescent dye according to [5], wherein the visible colored insoluble carrier particles are red-colored particles and the fluorescent dye is ATBTA-EU³⁺.
[7] The visible colored insoluble carrier particles labeled with a fluorescent dye according to any one of [1]-[6], wherein the insoluble carrier particles are polystyrene latex particles.
[8] An immunoassay method, comprising using the visible colored insoluble carrier particles labeled with a fluorescent dye according to any one of [1]-[7].
[9] The immunoassay method according to [8], further comprising determining the presence or absence of an antigen-antibody complex by:
   allowing the visible colored insoluble carrier particles labeled with a fluorescent dye according to any one of [1]-[7] to bind to an antigen-antibody complex having a target antigen or antibody; and
   visually observing coloring of the insoluble carrier particles and/or measuring fluorescence emitted by the insoluble carrier particles.
[10] The immunoassay method according to [8] or [9], which is an immunochromatographic method.
[11] An immunoassay kit including the visible colored insoluble carrier particles labeled with a fluorescent dye according to any one of [1]-[7].
[12] A method for preparing the visible colored insoluble carrier particles labeled with a fluorescent dye according to any one of [1]-[7], comprising:
   (i) a step of measuring absorbance spectra of visible colored insoluble carrier particles;
   (ii) a step of determining a wavelength range in which absorption by the visible colored insoluble carrier particles is low;
   (iii) a step of selecting a fluorescent dye that emits fluorescence in the determined wavelength range; and
   (iv) a step of labeling the visible colored insoluble carrier particles with the selected fluorescent dye.

### Advantageous Effects of Invention

The presence of the visible colored insoluble carrier particles labeled with a fluorescent dye of the present invention can be detected by visual observation or with the use of a fluorescence detector. Therefore, when the visible colored insoluble carrier particles labeled with a fluorescent dye of the present invention are used for immunoassay for the measurement of a target substance based on the presence of carrier particles, the presence or absence of the target substance can be determined by visual observation or with the use of a fluorescence detector. The use of the visible colored insoluble carrier particles labeled with a fluorescent dye of the present invention enables the implementation of POCT in a simple manner even in a facility or location in which no equipment such as a fluorescence detector is available. In addition, the use of a fluorescence detector also allows fluorescence measurement with improved accuracy.

### Brief Description of Drawings

Fig. 1 shows absorbance spectra of white and red polystyrene particles.
Fig. 2 shows absorbance spectra of white and blue polystyrene particles.
Fig. 3 shows absorbance spectra of white, green, and yellow polystyrene particles.

### Description of Embodiments

The present invention is described in detail below.

According to the present invention, visible colored insoluble carrier particles which are colored polystyrene latex particles to which carboxyl groups are introduced as a functional group and labeled with a fluorescent dye via the carboxyl groups, which are used for immunoassay, wherein the fluorescent dye used for labeling is a fluorescent substance that emits fluorescence with a wavelength of a color complementary to a color with a wavelength equivalent to an absorption wavelength of the visible colored insoluble carrier particles, are provided. The visible colored insoluble carrier particles according to the present invention are either blue-colored particles and the wavelength of fluorescence emitted by the fluorescent dye is 450 nm - 570 nm or the visible colored insoluble carrier particles according to the present invention are red-colored particles and the wavelength of fluorescence emitted by the fluorescent dye is 590 nm - 750 nm.

The term "insoluble carrier particles" refers to particles that are insoluble in liquid and serve as carriers capable of binding to and retaining an antigen or antibody. In the present invention, colored polystyrene latex particles used in the technical field of test agents or the like are used. The term "latex particles" refers to particles that are colloidally dispersed in water to form an emulsion. According to the present invention the material of the particles is polystyrene. However, materials used for solid-phase carriers in the technical field of test agents or the like for binding of a protein such as an antibody, an antigen, a ligand, or a receptor are also disclosed. Examples of such materials include: resins such as polystyrene, styrene copolymers (e.g., styrene-acrylic acid copolymer), polycarbonate, polymethylene methacrylate (PMMA), and polyvinyltoluene; silica; and cellulose. Alternatively, according to the present disclosure the particles may also be magnetic particles, the insides or surfaces of which are composed of a magnetic material such as iron, nickel, or cobalt.

The particle sizes of particles used herein are 10 nm to several hundreds of nanometers and preferably 30 nm - 500 nm.

The term "visible colored particles" refers to particles colored with a color that humans can visibly recognize. When the visible colored particles reflect visible light with a particular wavelength, humans can recognize their color by perceiving the reflected visible light. Visible light has a wavelength of approximately 380 nm - 750 nm. The color of colored particles is a hue expressed as a chromatic color which is, for example, red, orange, yellow, green, blue, or purple. Humans recognize coloring with each of these colors by perceiving the reflected visible light having the corresponding particular wavelengths below.
Red: 620-750 nm
Orange: 590-620 nm
Yellow: 570-590 nm
Green: 495-570 nm
Blue: 450-495 nm
Purple: 380-450 nm

Visible colored particles can be produced by staining the above particles with the relevant dyes. Upon staining with a dye, particle surfaces may be stained. However, in order to prevent desorption of a dye, it is preferable to stain the inside of each particle by impregnating particles with a dye. Although staining with a single dye is possible, different dyes may be used for staining to obtain visible colored particles colored with arbitrary color(s). For example, differently colored particles such as red particles, blue particles, green particles, yellow particles, and pink particles can be produced. Known dyes that are possible to use for staining of resin, etc. can be used for coloring. Coloring can be carried out using colorants or pigments such as Sudan Blue, Sudan Red III, Sudan Red IV, Quinizarin Green, and Oil Orange. In addition, differently colored particles made of different materials are commercially available. These commercially available colored particles also can be used.

According to the present invention, the visible colored insoluble carrier particles are colored polystyrene latex particles to which carboxyl groups are introduced as a functional group and labeled with a fluorescent dye via the carboxyl group. For example, a fluorescent dye is allowed to bind as a fluorescent labeling agent to visible colored insoluble carrier particles for labeling. The fluorescent dye is a fluorescent substance that emits fluorescence with a wavelength of a color complementary to a color with a wavelength equivalent to an absorption wavelength of the visible colored insoluble carrier particles, wherein the visible colored insoluble carrier particles are either blue-colored particles and the wavelength of fluorescence emitted by the fluorescent dye is 450 nm - 570 nm or the visible colored insoluble carrier particles are red-colored particles and the wavelength of fluorescence emitted by the fluorescent dye is 590 nm - 750 nm Note that a fluorescent dye is selected based on the fact that the absorption of fluorescence emitted by a fluorescent dye is low when fluorescence is absorbed by visible colored particles. This is because if fluorescence emitted by a fluorescent dye binding to visible colored particles is absorbed by the particles, fluorescence with a sufficient intensity cannot be measured in the form of signals from the particles. A state in which fluorescence emitted by a fluorescent dye is not absorbed by visible colored particles means a state in which the wavelength range of light absorbed by visible colored particles does not overlap with the wavelength range of fluorescence emitted by a fluorescent dye. For example, the absorbance spectra of visible colored particles to be used are measured using an absorptiometer so as to use a fluorescent dye emitting fluorescence with a wavelength range in which absorption of fluorescence by visible colored particles is low. The wavelength range in which absorption of fluorescence by visible colored particles is low is a wavelength range of a color complementary to a color with a wavelength range in which absorption of fluorescence by the visible colored particles is high. Therefore, it is possible to use a fluorescent substance that emits fluorescence with a wavelength of a color complementary to a color with a wavelength equivalent to the absorption wavelength of insoluble carrier particles. For example, red particles reflect red light, thereby allowing humans to visually recognize red coloring. Meanwhile, red colored particles absorb light of a color complementary to red. The wavelength of red light is approximately 620-750 nm. Colors complementary to red range from blue to green. The wavelength of blue light is around 450-495 nm and the wavelength of green light is around 495 - 570 nm. Therefore, when visible red-colored particles are used, a fluorescent dye that emits fluorescence with a wavelength of a color complementary to the color ranging from blue to green can be used. A color complementary to the color ranging from blue to green is a color ranging from orange to red with a wavelength around 590-750 nm. Accordingly, when visible red-colored insoluble carrier particles are used, a fluorescent dye that emits fluorescence with a wavelength range of 590-750 nm and preferably 590-650 nm is used.

In addition, blue particles reflect blue light and thus humans visually recognize blue coloring. Meanwhile, blue colored particles absorb light from a color complementary to blue. The wavelength of blue light is approximately 450-495 nm. Colors complementary to blue range from orange to red. The wavelength of orange light is around 590-620 nm and the wavelength of red light is around 620-750 nm. Therefore, when visible blue-colored particles are used, a fluorescent dye that emits fluorescence with a wavelength of a color complementary to a color ranging from orange to red can be used. A color complementary to a color ranging from orange to red is a color ranging from blue to green with a wavelength around 450-570 nm. Accordingly, when visible blue-colored insoluble carrier particles are used, a fluorescent dye that emits fluorescence with a wavelength range of 450-570 nm and preferably 500-550 nm is used. In addition, absorption at 600-700 nm tends to increase in the case of blue particles. In order to avoid this wavelength, a fluorescent dye that emits fluorescence with a wavelength range lower than 600-700 nm is used.

According to the present invention, the absorbance spectra of visible colored particles to be used are measured using an absorptiometer to determine the wavelength range of light of low absorption, a fluorescent dye that emits fluorescence with the relevant wavelength range is selected, and the fluorescent dye is allowed to bind to the visible colored particles by the method described below. With the use of such fluorescent dye, it is possible to reduce the absorption of fluorescence emitted by the fluorescent dye when fluorescence is absorbed by visible colored insoluble carrier particles, thereby achieving sufficient fluorescence intensity.

Examples of a fluorescent dye generally used for fluorescent labeling of visible colored particles include organic fluorescent dyes and derivatives of such fluorescent dyes having a basic structure comprising fluorescein, rhodamine, coumarin, Cy dye, Alexa Fluor (registered trademark), EvoBlue, oxazine, Carbopyronin, naphthalene, biphenyl, anthracene, phenenthrene, pyrene, carbazole, or the like. Also, a rare-earth complex such as europium (EU³⁺) chelate or terbium (Tb³⁺) chelate can be used. As an example of europium (EU³⁺) chelate, ATBTA ([4'-(4'-Amino-4-biphenylyl)-2,2':6',2"-terpyridine-6,6"-diylbis(methyliminodiacetato)]europate(III))-EU³⁺ having an amino group can be used. Further, a fluorescence protein such as a green fluorescent protein (GFP) can be used.

According to the present invention, labeling of visible colored particles with a fluorescent dye is carried out by introducing carboxyl groups as a functional group into the visible colored particles, also introducing a functional group into the fluorescent dye, and allowing the fluorescent dye to bind to the visible colored particles via the carboxyl groups. For example, it is possible to bind an amino group and a carboxyl group via an amide bond. According to the present disclosure, the functional groups may be bound to each other using a cross-linking reagent. Examples of a cross-linking reagent include N-hydroxysuccinimide ester, maleimide, EDAC(N-(3-dimethylaminopropyl)-N'-ethylcarbodiimide hydrochloride), and glutaraldehyde. In addition, if a rare-earth complex is used as a fluorescent dye, ATBTA-EU³⁺ or the like into which an amino group has been introduced can be used. Further, if a fluorescence protein is used, binding between functional groups may be used. In addition, according to the present disclosure a fluorescence protein can be bound to visible colored particles via physical adsorption. A commercially available fluorescent dye into which an amino group or a carboxyl group has been introduced can be used.

In another embodiment of the present disclosure, it is possible to premix a fluorescent dye with the main material of visible colored insoluble carrier particles. For example, insoluble carrier particles can be produced by kneading a fluorescent dye and a visible coloring dye into a material such as resin used for insoluble latex carrier particles. Further, fluorescent insoluble carrier particles can be labeled with a visible coloring dye. The fluorescent insoluble carrier particles used herein include insoluble carrier particles prepared with a substance capable of emitting fluorescence as well as insoluble carrier particles to which a fluorescent dye has been bound. Examples of such particles include particles comprising M^{I}M^{II}O₄ or M^{I}Al₅O₁₂ (where M^{I} denotes yttrium (Y), lanthanum (La), or gadolinium (Gd), and M^{II} denotes niobium (Nb), phosphorus (P), or vanadium (V)) activated with a rare earth element such as europium (Eu) or terbium (Tb). Those disclosed in JP Patent Publication (Kokai) No. 2012-32263 A can be used. In addition, the term "visible coloring dye" refers to a dye for coloring with a color that humans can visibly recognize. Known dyes used for staining of resin and the like can be used. Examples thereof include colorants and pigments such as Sudan Blue, Sudan Red III, Sudan Red IV, Quinizarin Green, and Oil Orange. For example, it is possible to produce insoluble carrier particles by kneading a fluorescent dye into resin that can be used as material for insoluble latex carrier particles and then stain the particles with a visible coloring dye. According to the present invention, visible colored insoluble carrier particles produced with the main material mixed with such fluorescent dyes and fluorescent insoluble carrier particles labeled with a visible coloring dye can also be used as visible colored insoluble carrier particles labeled with a fluorescent dye. That is, visible colored insoluble carrier particles labeled with a fluorescent dye include any insoluble carrier particles that are visible colored particles emitting fluorescence.

Several fluorescent dyes that can be used in the present invention are described below with the figures in parentheses corresponding to their fluorescence wavelengths (nm). Fluorescent dyes marked with ^{∗} are not according to the present invention. The fluorescence wavelengths may vary depending on measurement conditions. Measurement can be carried out within the range for each wavelength shown below ± several tens of nanometers (e.g., ± 25 nm). The present invention is not limited to the following fluorescent dyes. A variety of fluorescent dyes can be used in accordance with their fluorescence wavelengths.

### Organic fluorescent dyes

Dylight405 (420)^{∗}, DY-405 (420)^{∗}, Cascade Blue (420)^{∗}, Alexa Fluor405 (421)^{∗}, AMCA (440)^{∗}, Alexa-350 (442)^{∗}, AMCA-X (447)^{∗}, Pacific Blue (455), Marine Blue (460), DY-415 (467), Royal Blue (480), ATTO425 (484), Cy2 (505), ATTO465 (508), DY-475XL (509), NorthernLights493 (514), BODIPY 505/515 (515), DY-490 (516), Dylight488 (518), Alexa-488 (519), 5-FITC (519), FAM (520), DY-495-X5 (520), DY-495 (521), fluorescein (521), fluorescein isothiocyanate (FITC) (522), ATTO488 (523), HiLyte Flour488 (523), MFP488 (523), ATTO495 (527), OG-488 (524), Rhodamine110 (525), OG-514 (530), Oyster500 (530), Spectrum Green (538), Alexa-430 (541), ATTO520 (545), ATTO532 (553), Cas Y (554), Alexa-532 (554), DY-500XL (555), DY-485XL (560), Alexa-555 (565), HiLyte Plus 555 (552/567), DyLight549 (568), HiLyte Fluor555 (568), Cy3 (565), Dylight547 (570), Rhodamine (570), TRITC (570), DY548 (572)^{∗}, DY-554 (572)^{∗}, DY-555 (572)^{∗}, Alexa Fluor 546 (573)^{∗}, DY-556 (573)^{∗}, NorthernLights557 (574)^{∗}, Oyster550 (574)^{∗}, 5-TAMRA (574)^{∗}, DY-505-X5 (574)^{∗}, DY-547 (574)^{∗}, Oyster 556 (575)^{∗}, DY-549 (575)^{∗}, Alexa-546 (575)^{∗}, ATTO550 (576)^{∗}, PE (578)^{∗}, B-PE (578)^{∗}, R-PE (578)^{∗}, DY-560 (578)^{∗}, TAMRA (580)^{∗}, Tetramethyl rhodamine (578)^{∗}, RITC/TMR (580)^{∗}, MFP555 (585)^{∗}, Spectrum Orange (588)^{∗}, DY-510XL (590), Rhodamine B (590), ATTO565 (592), Cy3.5 (596), Rhodamine Red X (ROX) (599), DY-590 (599), 5-ROX (602), Alexa-568 (603), BODIPY 580/605 (605), Spectrum Red (612), ECD (613), Texas Red (615), DyLight594 (618), Alexa Flour594 (619), HiLyte Fluor TR (622), ATTO590 (624), MFP590 (624), DY-610 (630), DY480XL (630), ATTO610 (634), DY-615 (641), C-Phycocyanin (647), ATTO620 (643), Alexa-633 (647), Phycocianin (650), DY-481XL (650), ATTO633 (657), DY-630 (657), DY-632 (657), DY-633 (657), MFP631 (658), DyLight633 (658), NorthernLights637 (658), DY-631 (658), DY-634 (658), Allophycocyanin (APC) (660), APC-XL (660), DY-520XL (664), Alexa-647 (668), Cy5 (667), DY-521XL (668), Oyster645 (669), Quantum Red (670), DY-635 (671), DY-636 (671), DY-647 (673), DyLight647 (673), HiLyte Fluor (647), DyLight649 (674), HiLyte Plus647 (674), Oyster650 (674), DY-648 (674), DY-650 (674), PerCP (675), DY-652 (675), DY-649 (676), DY-651 (678), Oyster656 (679), ATTO655 (684), Alexa-660 (690), Cy5.5 (694), DY-677 (694), DY-678 (698), HiLyte Fluor680 (699), DY-675 (699), DY-676 (699), IRDye700DX (700), Alexa-680 (702), DY-681 (708), DY-680 (709), DY-682 (709), DyLight680 (715), Alexa Fluor700 (723), DY-700 (730), DY-701 (731), DY-730 (758)^{∗}, DY-732 (759)^{∗}, DY-734 (759)^{∗}, DY-731 (760)^{∗}, DY-752 (772)^{∗}, DY-750 (776)^{∗}, DyLight750 (778)^{∗}, HiLyte Fluor750 (778)^{∗}, DY-749 (778)^{∗}, HiLyte Plus750 (779)^{∗}, APC7 (779)^{∗}, DY-751 (779)^{∗}, DyLight800 (794)^{∗}, IRDye800CW (800)^{∗}, DY-780 (800)^{∗}, DY-781 (800)^{∗}, DY-782 (800)^{∗}, DY-776 (801)^{∗}, DY-777 (801)^{∗}, IRDye800 (806)^{∗}, etc.

### Rare-earth complex

ATBTA-EU³⁺ (616), etc.

### Fluorescence proteins

Sirius (424)^{∗}, CFP (505), AcGFP (505), EGFP (509), EYFP (527), YFP (527), ZsYellow (539), mOrange (562), DsRed2 (582)^{∗}, AsRed2 (592), mRFP1 (607), mCherry (610), HcRed (618), mRasberry (625), mPlum (649), etc.

For example, when blue polystyrene latex particles are used as visible colored particles, since blue polystyrene latex particles have an absorption peak around 600-700 nm, a fluorescent dye having a fluorescence wavelength within a wavelength range of, for example, less than 600-700 nm, such as 450-570 nm according to the present invention, in particular approximately 450-550 nm and preferably approximately 500 nm, can be used. An example of such a fluorescent dye is fluorescein with a fluorescence wavelength of 521 nm. In addition, when red polystyrene latex particles are used as visible colored particles, since red polystyrene latex particles have an absorption peak around 500-550 nm, a fluorescent dye having a fluorescence wavelength that is, for example, greater than 500-550 nm, such as 590-750 nm according to the present invention, in particular approximately 600-700 nm, can be used. An example of such fluorescent dye is ATBTA-EU³⁺ with a fluorescence wavelength of 616 nm.

The visible colored insoluble carrier particles labeled with a fluorescent dye of the present invention can be visually recognized by humans, and the fluorescent dye emits fluorescence. When insoluble carrier particles are used for immunoassay, an antigen or antibody is allowed to bind to insoluble carrier particles, and an antibody or antigen contained in a test subject's sample is allowed to bind thereto, followed by collection or accumulation of insoluble carrier particles having a complex of the antigen and the antibody. The presence of the antibody or antigen in the test subject's sample is detected based on the presence or absence of such insoluble carrier particles. When the visible colored insoluble carrier particles labeled with a fluorescent dye of the present invention are used for immunoassay, the presence of the antibody or antigen can be detected visually through visual observation of color. In addition, the presence of the antibody or antigen can be detected by measurement of fluorescence emitted by the insoluble carriers using a fluorescence detector.

In order to allow an antigen or antibody to bind to the visible colored insoluble carrier particles labeled with a fluorescent dye, it is possible to cause binding between a functional group of an insoluble carrier particle and a functional group of an antigen or antibody in a manner similar to the method for allowing a fluorescent dye to bind to insoluble carrier particles. In addition, it is also possible to allow an antigen or antibody to bind to insoluble carrier particles via physical adsorption.

The present invention further encompasses a method for preparing the above visible colored insoluble carrier particles labeled with a fluorescent dye, comprising: (i) a step of measuring absorbance spectra of the visible colored insoluble carrier particles; (ii) a step of determining a wavelength range in which absorption by the visible colored insoluble carrier particles is low; (iii) a step of selecting a fluorescent dye that emits fluorescence in the determined wavelength range; and (iv) a step of labeling the visible colored insoluble carrier particles with the selected fluorescent dye.

Immunoassay with the use of the visible colored insoluble carrier particles labeled with a fluorescent dye of the present invention is not limited. It may be an immunochromatographic method, an agglutination method, or the like and it is preferably an immunochromatographic method. An immunochromatographic method can be carried out using a known immunochromatographic device by a known method. In addition, the present invention encompasses an immunoassay method with the use of visible colored insoluble carrier particles labeled with a fluorescent dye. The method further comprises determining the presence or absence of an antigen-antibody complex by: allowing the visible colored insoluble carrier particles labeled with a fluorescent dye to bind to an antigen-antibody complex having a target antigen or antibody; and visually observing coloring of the insoluble carrier particles and/or measuring fluorescence emitted by the insoluble carrier particles. That is, an antigen or antibody is subjected to measurement based on the color or fluorescence of visible colored insoluble carrier particles labeled with a fluorescent dye. Measurement includes qualitative measurement and quantitative measurement.

For example, in an immunochromatographic method for detecting an antigen in a test subject's sample, an antibody capable of specifically binding to a target substance, such as an antigen, is immobilized at a predetermined site (detection site) on an appropriate solid-phase substrate such as a nitrocellulose membrane. A complex of the target substance with visible colored insoluble carrier particles labeled with the fluorescent dye of the present invention, which have been bound to an antigen to which an antibody to be detected specifically binds, is allowed to migrate and developed on the solid-phase substrate with the use of a capillary phenomenon. Here, after the insoluble carrier particles and the target substance are mixed, the mixture can be added to a predetermined site on the solid-phase substrate (a sample addition site). Alternatively, the insoluble carrier particles can be immobilized on the solid-phase substrate (at a labeling site) in advance, thereby allowing dissolution of the insoluble carrier particles with the addition of a sample liquid so as to allow the insoluble carrier particles to form a complex with the target substance. As a result, a complex of the solid-phased antigen - the antibody as a target substance - the visible colored insoluble carrier particles labeled with a fluorescent dye is formed at the detection site on the solid-phase substrate. The target substance can be detected by confirming the presence of the visible colored insoluble carrier particles labeled with a fluorescent dye at the detection site through visual observation or measurement with the use of a fluorescence detector. For measurement of an antibody contained in the test subject's sample, an antigen to which the antibody as a target substance specifically binds is immobilized on a solid-phase substrate, and then visible colored insoluble carrier particles labeled with a fluorescent dye are allowed to bind to the antigen.

A target substance for the method of the present invention is not limited. Examples thereof include: viruses such as HIV, hepatitis virus, and influenza virus; and bacteria such as *Staphylococcus aureus, Diplococcus pneumonia,* and enterohemorrhagic *Escherichia coli.* The sample is also not limited. Examples of a sample that can be used include pharynx or nasal swab, pharynx or nasal lavage fluid, nasal aspirate, saliva, serum, plasma, feces, fecal suspension, and urine collected from a test subject, as well as culture solutions thereof.

Moreover, the present invention encompasses an immunoassay kit for detection, which includes visible colored insoluble carrier particles labeled with a fluorescent dye according to the present invention to which an antigen or antibody has been bound as a target substance. The kit further includes an immunochromatographic device and a brochure of the device.

### Examples

The present invention will be more specifically described by way of the following Examples; however, the present invention is not limited to Examples. In the Examples, the symbol "%" means "% by weight."

### Example 1: Preparation of visible colored fluorescent particles labeled with a fluorescent dye with the use of colored polystyrene particles having a carboxyl group as a functional group

### 1. Binding of a europium chelate fluorescent labeling agent

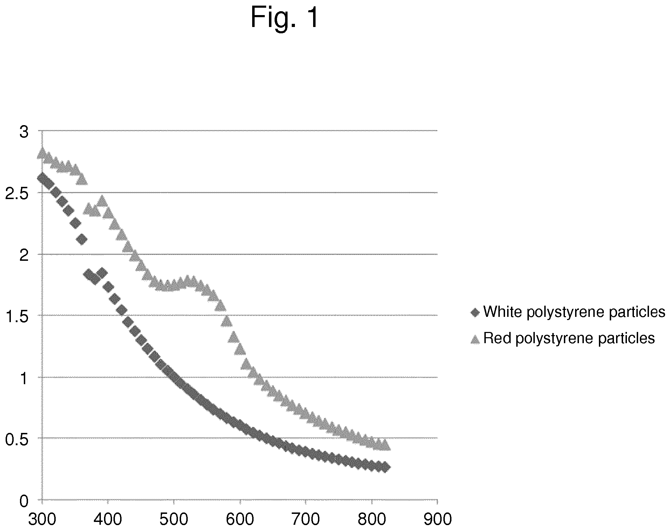

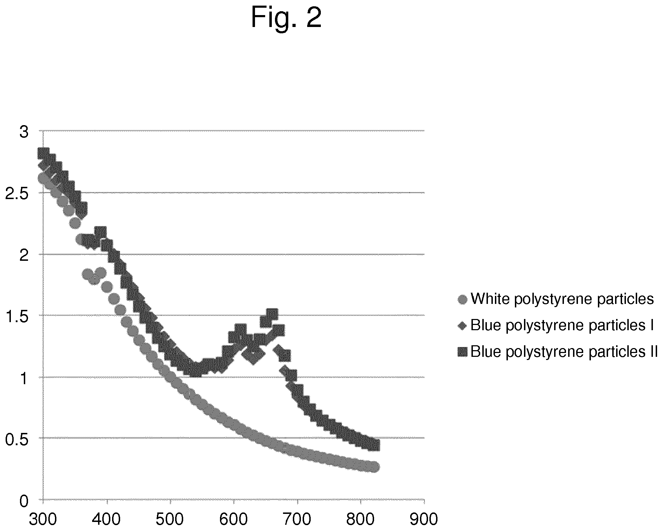

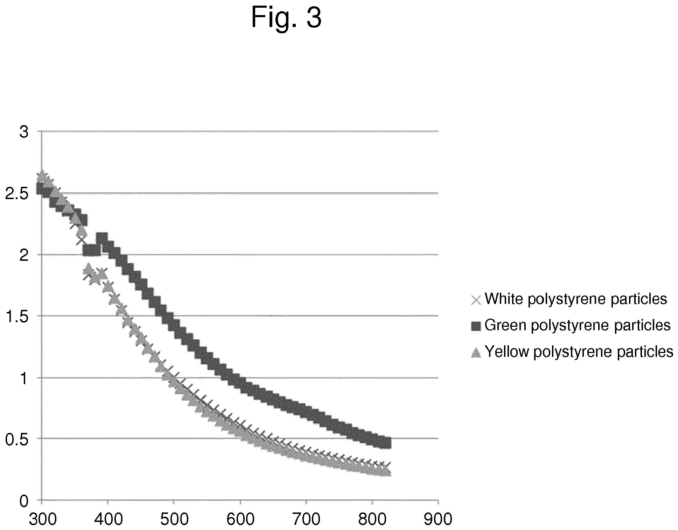

Different color types of visible colored polystyrene particles having a carboxyl group as a functional group (see Table 1 and Figs. 1-3) were diluted with a 10 mM HEPES (pH 7.0) buffer solution to obtain a concentration of 0.1%. ATBTA-Eu³⁺ (Tokyo Chemical Industry Co., Ltd.) serving as a europium chelate fluorescent labeling agent was added thereto to obtain a concentration of 0.1 mg/mL, followed by stirring. Thereafter, carbodiimide was added thereto to obtain a concentration of 1%, followed by stirring. The resultant was allowed to react at room temperature for 1 hour, followed by centrifugation at 7000 g for 30 minutes. Thereafter, the supernatant was removed and the resultant was resuspended in a solution containing 50 mM Tris (pH 9.0) and 3% BSA.

### 2. Binding of an aminofluorescein fluorescent labeling agent

Different color types of visible colored polystyrene particles having a carboxyl group as a functional group (see Table 1 and Figs. 1-3) were diluted with a 10 mM HEPES (pH 7.0) buffer solution to obtain a concentration of 0.1%. 6-Aminofluorescein (Tokyo Chemical Industry Co., Ltd.) was added thereto to obtain a concentration of 0.1 mg/mL, followed by stirring. Thereafter, carbodiimide was added thereto to obtain a concentration of 1%, followed by stirring. The resultant was allowed to react at room temperature for 1 hour, followed by centrifugation at 7000 g for 30 minutes. Thereafter, the supernatant was removed and the resultant was resuspended in a solution containing 50 mM Tris (pH 9.0) and 3% BSA.

Table 1 lists the visible colored polystyrene latex particles used herein. Figs. 1-3 show absorbance spectra of the respective particles.

**[Table 1] Different color types of visible colored polystyrene particles. Visible colored polystyrene particles marked with ∗ are not according to the present invention.**

| Sample | ϕ (µm) |
|---|---|
| White latex^{∗} | 0.319 |
| Red latex | 0.4 |
| Blue latex I | 0.404 |
| Blue latex II | 0.4 |
| Green latex^{∗} | 0.479 |
| Yellow latex^{∗} | 0.3 |

### 3. Measurement of the fluorescence intensity of polystyrene particles bound to the europium chelate fluorescent labeling agent

The polystyrene particles prepared in 1 above to which the europium chelate fluorescent labeling agent had been bound were diluted with a solution containing 50 mM Tris (pH 9.0) and 3% BSA to obtain a concentration of 0.01%. The resulting solution was added to wells of a 96-well FluoroNunc plate (100 µL each). The fluorescence intensity was measured using a fluorescence plate reader (MTP-650FA; CORONA ELECTRIC Co., Ltd.) at 340 nm ex/615 nm em. As controls, a solution containing 50 mM Tris (pH 9.0) and 3% BSA and visible colored polystyrene particles to which the europium chelate fluorescent labeling agent had not been bound were subjected to measurement.

As a result, the visible colored polystyrene particles to which the europium chelate fluorescent labeling agent had not been bound showed intensity substantially comparable to or significantly lower than the intensity exhibited by the solution containing 50 mM Tris (pH 9.0) and 3% BSA. In the case of the visible colored polystyrene particles to which the europium chelate fluorescent labeling agent had been bound, the fluorescence intensity was 300 or higher for each of red polystyrene particles and yellow polystyrene particles showing low absorption around 615 nm on the absorbance spectrum, the fluorescence intensity was about 160 for green polystyrene particles, and the fluorescence intensity was as low as 100 or lower for two types of blue polystyrene particles showing strong absorption around 615 nm. Table 2 lists the results.

**[Table 2] Europium chelate fluorescence intensity measurement using a fluorescence plate reader**

| Sample | Control | Fluorescence intensity |
|---|---|---|
| White polystyrene particles^{∗} | 0.709 | 1652 |
| Red polystyrene particles | 1.206 | 354.6 |
| Blue polystyrene particles I | 0.866 | 91.53 |
| Blue polystyrene particles II | 1.152 | 88.3 |
| Green polystyrene particles^{∗} | 0.902 | 169.3 |
| Yellow polystyrene particles^{∗} | 0.943 | 590.8 |
| 50 mM Tris (pH 9.0), 3%BSA | 0.759 | - |

### 4. Measurement of the fluorescence intensity of polystyrene particles bound to the fluorescein fluorescent labeling agent

The polystyrene particles prepared in 2 above to which fluorescein fluorescent labeling agent had been bound were diluted with a solution containing 50 mM Tris (pH 9.0) and 3% BSA to obtain a concentration of 0.05%. The resulting solution was added to wells of a 96-well FluoroNunc plate (100 µL each). The fluorescence intensity was measured using a fluorescence plate reader (MTP-650FA; CORONA ELECTRIC Co., Ltd.) at 490 nm ex/530 nm em. As controls, a solution containing 50 mM Tris (pH 9.0) and 3% BSA, visible colored polystyrene particles mixed only with to which the fluorescein fluorescent labeling agent had not been bound were subjected to measurement.

As a result, the visible colored polystyrene particles to which the fluorescein fluorescent labeling agent had not been bound showed intensity substantially comparable to or significantly lower than the intensity exhibited by the solution containing 50 mM Tris (pH 9.0) and 3% BSA. In the case of the visible colored polystyrene particles to which the fluorescein fluorescent labeling agent had been bound, the fluorescence intensity was 500 or higher for yellow polystyrene particles showing low absorption around 530 nm on the absorbance spectrum, the fluorescence intensity was about 170 for green polystyrene particles showing low absorption (greater than that of yellow polystyrene particles), the fluorescence intensity was 70 or higher for two types of blue polystyrene particles showing low absorption (greater than that of green polystyrene particles), and the fluorescence intensity was low for red polystyrene particles showing strong absorption around 530 nm. Table 3 lists the results.

**[Table 3] Fluorescein fluorescence intensity measurement using a fluorescence plate reader**

| Sample | Control | Fluorescence intensity |
|---|---|---|
| White polystyrene particles^{∗} | 0.047 | 879.2 |
| Red polystyrene particles | 0.05 | 25.37 |
| Blue polystyrene particles I | 0.056 | 115.4 |
| Blue polystyrene particles II | 0.111 | 76.36 |
| Green polystyrene particles^{∗} | 0.064 | 171.3 |
| Yellow polystyrene particles^{∗} | 0.056 | 506.8 |
| 50 mM Tris (pH 9.0), 3%BSA | 0.058 | - |

The following were revealed from the above results for the absorbance spectra and fluorescence intensities for colored polystyrene particles to which the europium chelate fluorescent labeling agent and the aminofluorescein fluorescent labeling agent had been bound: fluorescence can be efficiently detected by allowing a fluorescent labeling agent to bind to insoluble carrier particles when the fluorescent labeling agent is allowed to bind to insoluble carrier particles showing low absorption of the fluorescence wavelength of the fluorescent substance; and fluorescence measurement cannot be performed with high efficiency with the use of insoluble carrier particles showing strong absorption of the same.

### Example 2: Binding of an anti-influenza A virus NP antibody and a fluorescent labeling agent to visible colored polystyrene particles having a carboxyl group as a functional group and detection of an influenza A virus NP antigen through immunochromatography based on such binding

In Example 2, visible colored polystyrene particles were compared. Verification was carried out using, as materials, red polystyrene particles and blue polystyrene particles I, both of which showed high visibility.

### 1. Preparation of anti-influenza A virus NP monoclonal antibody

A BALB/c mouse was immunized with an influenza A virus antigen and raised for a premeasured time. The spleen was excised out from the mouse and the spleen cells were fused with mouse myeloma cells (P3 × 63) in accordance with the Kohler's method (Kohler et al., Nature, vol. 256, p495-497 (1975)). The resultant hybridoma cells were kept in an incubator of 37°C. While the antibody activity of the supernatant was checked by ELISA using a plate on which an influenza A virus NP antigen was immobilized, the cells were purified (monocloned). Two cell strains were obtained and separately administered intraperitoneally to BALB/c mice treated with pristane. About two weeks later, ascites fluids containing the antibody were collected.

IgG was purified from each of the obtained ascites fluids by use of affinity chromatography using a protein A column to obtain two types of purified anti-influenza A virus NP antibodies.

### 2. Immobilization of anti-influenza A virus NP antibody on nitrocellulose membrane

The purified anti-influenza A virus NP antibody was diluted with purified water so as to obtain a concentration of 1.0 mg/mL. The diluted solution was linearly applied to a premeasured position on a nitrocellulose membrane (Unisart CN 95; Sartorius Stedim Biotech) backed with a PET film. The nitrocellulose membrane was dried at 45°C for 30 minutes to obtain an anti-influenza A virus NP antibody immobilized membrane (hereinafter referred to as an antibody immobilized membrane).

### 3. Immobilization of anti-influenza A virus NP antibody and a fluorescent labeling substance to visible colored polystyrene particles

Another purified anti-influenza A virus NP antibody, which was not used for immobilization to a nitrocellulose membrane, was diluted with purified water so as to obtain a concentration of 1.0 mg/mL. To this, red polystyrene particles or blue polystyrene particles I were added so as to obtain a concentration of 0.1%, followed by stirring. Thereafter, ATBTA-Eu³⁺ (Tokyo Chemical Industry Co., Ltd.) serving as a europium chelate fluorescent labeling agent was added to the liquid mixture of the red polystyrene particles and the antibody to obtain a concentration of 0.1 mg/mL. Meanwhile, 6-aminofluorescein (Tokyo Chemical Industry Co., Ltd.) was added to the liquid mixture of the blue polystyrene particles I and the antibody to obtain a concentration of 0.1 mg/mL, followed by stirring. Carbodiimide was added to each liquid mixture to obtain a concentration of 1%, followed by stirring. The resultant was allowed to react at room temperature for 1 hour, followed by centrifugation at 7000 g for 30 minutes. Thereafter, the supernatant was removed and the resultant was resuspended in a solution containing 50 mM Tris (pH 9.0) and 3% BSA to obtain anti-influenza A virus NP antibody + red polystyrene particles bound to europium chelate and anti-influenza A virus antibody + blue polystyrene particles bound to fluorescein.

### 4. Detection of influenza A virus NP antigen by immunochromatography

The antibody-immobilized membrane obtained in 2 was attached to a backing sheet, an absorption band, a conjugate pad, and a sample pad to obtain a test piece.

The anti-influenza A virus NP antibody + red polystyrene particles bound to europium chelate and anti-influenza A virus antibody + blue polystyrene particles bound to fluorescein obtained in 3 above were diluted with a solution containing 50 mM Tris (pH 9.0) and 3% BSA to obtain a concentration of 0.1%. Thus, diluted red particles and diluted blue particles were obtained.

An antigen dilution solution was prepared by diluting the influenza A virus NP antigen with a specimen suspension (10 mM Tris (pH7.0), 3%BSA, 0.2% TRITON® X-100).

The antigen dilution solution (100 µL) was mixed with diluted red particles (5 µL) or diluted blue particles (5 µL). The total amount of each mixture was added dropwise to a test piece sample pad. Each sample pad was allowed to stand still for 15 minutes. Then, the presence or absence of test lines was visually determined. Further, fluorescence was visually confirmed with a UV transilluminator (Benchtop 2UV transilluminator; UVP). Comparison and investigation of the detection limit between the diluted red particles and red polystyrene particles used as a control to which the anti-influenza A virus NP antibody alone had been bound were carried out.

As a result, no test line was confirmed for the test piece tested with a specimen suspension containing no antigen through visual observation or detection of fluorescence with the use of the UV transilluminator. Meanwhile, for the test piece tested with a specimen suspension containing the antigen, a test line for coloring was visually confirmed, and a test line for fluorescence was confirmed with the use of the UV transilluminator. Table 4 lists the results.

**[Table 4] Results of detection of influenza A virus NP antigen**

| | Without antigen | | With antigen | |
|---|---|---|---|---|
| | Visual observation of coloring | Visual observation of fluorescence | Visual observation of coloring | Visual observation of fluorescence |
| Diluted red particles | -- | -- | + | + |
| Diluted blue particles | -- | -- | + | + |

Further, as a result of comparison of and investigation regarding the detection limit between the red polystyrene particles to which the anti-influenza A virus NP antibody alone had been bound and the diluted red particles, no change in the detection limit was confirmed through visual observation of coloring. The sensitivity was found to be two times that of the control upon visual observation of fluorescence. Table 5 shows the results.

**[Table 5] Comparison of detection limit**

| | Factor of dilution of antieen | | | | | | | |
|---|---|---|---|---|---|---|---|---|
| | × 2 | | × 4 | | × 8 | | × 16 | |
| | Visual observation of coloring | Visual observation of fluorescence | Visual observation of coloring | Visual observation of fluorescence | Visual observation of coloring | Visual observation of fluorescence | Visual observation of coloring | Visual observation of fluorescence |
| Control red particles | + | | + | - | - | | - | |
| Diluted red particles | + | + | + | + | | + | - | - |

The above results revealed that visual confirmation of a test line for coloring and measurement of a test line for fluorescence with the use of an apparatus can be carried out by applying fluorescent-substance-bound colored particles prepared by the above method to immunochromatography. In the above Examples, fluorescence is visually confirmed. Meanwhile, fluorescence can be detected with improved sensitivity with the use of the apparatus.

### Industrial Applicability

The visible colored insoluble carrier particles labeled with a fluorescent dye of the present invention can be used for immunoassay such as immunochromatography.

## Claims

1. Visible colored insoluble carrier particles which are colored polystyrene latex particles to which carboxyl groups are introduced as a functional group and labeled with a fluorescent dye via the carboxyl groups, which are used for immunoassay, wherein the fluorescent dye used for labeling is a fluorescent substance that emits fluorescence with a wavelength of a color complementary to a color with a wavelength equivalent to an absorption wavelength of the visible colored insoluble carrier particles, the visible colored insoluble carrier particles are blue-colored particles and the wavelength of fluorescence emitted by the fluorescent dye is 450 nm - 570 nm.

2. The visible colored insoluble carrier particles labeled with a fluorescent dye according to claim 1, wherein the visible colored insoluble carrier particles are blue-colored particles and the fluorescent dye is fluorescein or fluorescein isothiocyanate.

3. Visible colored insoluble carrier particles which are colored polystyrene latex particles to which carboxyl groups are introduced as a functional group and labeled with a fluorescent dye via the carboxyl groups, which are used for immunoassay, wherein the fluorescent dye used for labeling is a fluorescent substance that emits fluorescence with a wavelength of a color complementary to a color with a wavelength equivalent to an absorption wavelength of the visible colored insoluble carrier particles, the visible colored insoluble carrier particles are red-colored particles and the wavelength of fluorescence emitted by the fluorescent dye is 590 nm - 750 nm.

4. The visible colored insoluble carrier particles labeled with a fluorescent dye according to claim 3, wherein the visible colored insoluble carrier particles are red-colored particles and the fluorescent dye is ATBTA-EU³⁺.

5. An immunoassay method, comprising using the visible colored insoluble carrier particles labeled with a fluorescent dye according to any one of claims 1-4.

6. The immunoassay method according to claim 5, further comprising determining the presence or absence of an antigen-antibody complex by:
allowing the visible colored insoluble carrier particles labeled with a fluorescent dye according to any one of claims 1-4 to bind to an antigen-antibody complex having a target antigen or antibody; and
visually observing coloring of the insoluble carrier particles and/or measuring fluorescence emitted by the insoluble carrier particles.

7. The immunoassay method according to claim 5 or 6, which is an immunochromatographic method.

8. An immunoassay kit including the visible colored insoluble carrier particles labeled with a fluorescent dye according to any one of claims 1-4.

9. A method for preparing the visible colored insoluble carrier particles labeled with a fluorescent dye according to any one of claims 1-4, comprising:
(i) a step of measuring absorbance spectra of visible colored insoluble carrier particles;
(ii) a step of determining a wavelength range in which absorption by the visible colored insoluble carrier particles is low;
(iii) a step of selecting a fluorescent dye that emits fluorescence in the determined wavelength range; and
(iv) a step of labeling the visible colored insoluble carrier particles with the selected fluorescent dye.

## Patentansprüche

1. Farbige, unlösliche Trägerteilchen, die gefärbte Polystyrol-Latexteilchen sind, an die Carboxylgruppen als funktionelle Gruppe eingeführt sind und die über die Carboxylgruppen mit einem Fluoreszenzfarbstoff markiert sind, die für Immunoassays verwendet werden, wobei der zur Markierung verwendete Fluoreszenzfarbstoff eine fluoreszierende Substanz ist, die Fluoreszenz mit einer Wellenlänge einer Farbe emittiert, die komplementär zu einer Farbe mit einer Wellenlänge ist, die einer Absorptionswellenlänge der farbigen, unlöslichen Trägerteilchen entspricht, wobei die farbigen, unlöslichen Trägerteilchen blau gefärbte Teilchen sind und die Wellenlänge der von dem Fluoreszenzfarbstoff emittierten Fluoreszenz 450 nm - 570 nm beträgt.

2. Farbige, unlösliche Trägerteilchen markiert mit einem Fluoreszenzfarbstoff nach Anspruch 1, wobei die farbigen, unlöslichen Trägerteilchen blau gefärbte Teilchen sind und der Fluoreszenzfarbstoff Fluorescein oder Fluoresceinisothiocyanat ist.

3. Farbige, unlösliche Trägerteilchen, die gefärbte Polystyrol-Latexteilchen sind, an die Carboxylgruppen als funktionelle Gruppe eingeführt sind und die über die Carboxylgruppen mit einem Fluoreszenzfarbstoff markiert sind, die für Immunoassays verwendet werden, wobei der zur Markierung verwendete Fluoreszenzfarbstoff eine fluoreszierende Substanz ist, die Fluoreszenz mit einer Wellenlänge einer Farbe emittiert, die komplementär zu einer Farbe mit einer Wellenlänge ist, die einer Absorptionswellenlänge der farbigen, unlöslichen Trägerteilchen entspricht, wobei die farbigen, unlöslichen Trägerteilchen rot gefärbte Teilchen sind und die Wellenlänge der von dem Fluoreszenzfarbstoff emittierten Fluoreszenz 590 nm - 750 nm beträgt.

4. Farbige, unlösliche Trägerteilchen markiert mit einem Fluoreszenzfarbstoff nach Anspruch 3, wobei die farbigen, unlöslichen Trägerteilchen rot gefärbte Teilchen sind und der Fluoreszenzfarbstoff ATBTA-EU³⁺ ist.

5. Immunoassay-Verfahren, umfassend die Verwendung der farbigen, unlöslichen Trägerteilchen markiert mit einem Fluoreszenzfarbstoff nach einem der Ansprüche 1-4.

6. Immunoassay-Verfahren nach Anspruch 5, ferner umfassend die Bestimmung der Anwesenheit oder Abwesenheit eines Antigen-Antikörper-Komplexes durch:
Bindenlassen der farbigen unlöslichen Trägerteilchen markiert mit einem Fluoreszenzfarbstoff nach einem der Ansprüche 1-4, an einen Antigen-Antikörper-Komplex mit einem Zielantigen oder -antikörper; und
visuelle Beobachtung der Färbung der unlöslichen Trägerteilchen und/oder Messung der von den unlöslichen Trägerteilchen emittierten Fluoreszenz.

7. Immunoassay-Verfahren nach Anspruch 5 oder 6, das ein immunchromatographisches Verfahren ist.

8. Immunoassay-Kit, das die farbigen, unlöslichen Trägerteilchen markiert mit einem Fluoreszenzfarbstoff nach einem der Ansprüche 1 bis 4 enthält.

9. Verfahren zur Herstellung der farbigen, unlöslichen Trägerteilchen markiert mit einem Fluoreszenzfarbstoff nach einem der Ansprüche 1-4 umfassend:
(i) einen Schritt des Messens von Absorptionsspektren von farbigen, unlöslichen Trägerteilchen,
(ii) einen Schritt des Bestimmens eines Wellenlängenbereichs, in dem die Absorption der sichtbaren gefärbten, unlöslichen Trägerteilchen gering ist,
(iii) einen Schritt des Auswählens eines Fluoreszenzfarbstoffs, der in dem bestimmten Wellenlängenbereich Fluoreszenz emittiert, und
(iv) einen Schritt des Markierens der farbigen, unlöslichen Trägerteilchen mit dem ausgewählten Fluoreszenzfarbstoff.

## Revendications

1. Particules de support insolubles colorées qui sont des particules de latex de polystyrène colorées auxquelles des groupes carboxyle sont introduits en tant que groupe fonctionnel et marquées avec un colorant fluorescent via les groupes carboxyle, qui sont utilisées pour un dosage immunologique, dans lequel le colorant fluorescent utilisé pour le marquage est une substance fluorescente qui émet une fluorescence avec une longueur d'onde d'une couleur complémentaire à une couleur avec une longueur d'onde équivalente à une longueur d'onde d'absorption des particules de support insolubles colorées , les particules de support insolubles colorées sont des particules de couleur bleue et la longueur d'onde de la fluorescence émise par le colorant fluorescent est de 450 nm - 570 nm.

2. Particules de support insolubles colorées qui sont marquées avec un colorant fluorescent selon la revendication 1, dans lesquelles les particules de support insolubles colorées sont des particules de couleur bleue et le colorant fluorescent est la fluorescéine ou l'isothiocyanate de fluorescéine.

3. Particules de support insolubles colorées qui sont des particules de latex de polystyrène colorées auxquelles des groupes carboxyle sont introduits en tant que groupe fonctionnel et marquées avec un colorant fluorescent via les groupes carboxyle, qui sont utilisées pour un dosage immunologique, dans lequel le colorant fluorescent utilisé pour le marquage est une substance fluorescente qui émet une fluorescence avec une longueur d'onde d'une couleur complémentaire à une couleur avec une longueur d'onde équivalente à une longueur d'onde d'absorption des particules de support insolubles colorées , les particules de support insolubles colorées sont des particules de couleur rouge et la longueur d'onde de la fluorescence émise par le colorant fluorescent est de 590 nm - 750 nm.

4. Particules de support insolubles colorées qui sont marquées avec un colorant fluorescent selon la revendication 3, dans lesquelles les particules de support insolubles colorées sont des particules de couleur rouge et le colorant fluorescent est ATBTA-EU³⁺.

5. Procédé de dosage immunologique, comprenant l'utilisation des particules de support insolubles colorées qui sont marquées avec un colorant fluorescent selon l'une quelconque des revendications 1 à 4.

6. Procédé de dosage immunologique selon la revendication 5, comprenant en outre la détermination de la présence ou de l'absence d'un complexe antigène-anticorps par :
permettant aux particules de support insolubles colorées qui sont marquées avec un colorant fluorescent selon l'une quelconque des revendications 1 à 4 de se lier à un complexe antigène-anticorps ayant un antigène ou un anticorps cible ; et
observer visuellement la coloration des particules de support insolubles et/ou mesurer la fluorescence émise par les particules de support insolubles.

7. Procédé de dosage immunologique selon la revendication 5 ou 6, qui est une méthode immunochromatographique.

8. Kit de dosage immunologique contenant les particules de support insolubles colorées qui sont marquées avec un colorant fluorescent selon l'une quelconque des revendications 1 à 4.

9. Procédé de préparation des particules de support insolubles colorées qui sont marquées avec un colorant fluorescent selon l'une quelconque des revendications 1 à 4, comprenant :
(i) une étape de mesure des spectres d'absorbance des particules de support insolubles colorées ;
(ii) une étape de déterminer une gamme de longueurs d'onde dans laquelle l'absorption par les particules de support insolubles colorées est faible ;
(iii) une étape de sélection d'un colorant fluorescent qui émet une fluorescence dans la gamme de longueurs d'onde déterminée ; et
(iv) une étape de marquage des particules de support insolubles colorées avec le colorant fluorescent sélectionné.
